(19) Europäisches Patentamt
European Patent Office
Office européen des brevets

(11) Veröffentlichungsnummer: **0 147 741**
**B1**

# (12) EUROPÄISCHE PATENTSCHRIFT

(45) Veröffentlichungstag der Patentschrift: **02.08.89**

(51) Int. Cl.⁴: **A 61 K 31/685,** C 07 F 9/10

(21) Anmeldenummer: **84115401.6**

(22) Anmeldetag: **13.12.84**

ERRATUM

| DIE TEXTSTELLE :<br>TEXT PUBLISHED :<br>LE PASSAGE SUIVANT : | (SEITE, SPALTE, ZEILE)<br>(PAGE, COLUMN, LINE)<br>(PAGE, COLONNE, LIGNE) | | LAUTET BERICHTIGT:<br>SHOULD READ :<br>DEVRAIT ETRE LU : |
|---|---|---|---|
| 463 - 469; 19B3, 54 (2) | 2 | 44 | 463 - 469; 1983, 54 (2) |
| 75 bis 36 Gew.-% der | 3 | 38 | 75 bis 86 Gew.-% der |
| Menge nach 30 Minuten | 6 | 46 | PL-Menge nach 30 Minuten |
| OPPI = | 8 | 6 | DPPI = |
| 80 Gew.-% 1 2-Diacyl | 9 | 22 | 80 Gew.-% 1,2-Diacyl |
| Beispie1 14 | 10 | 33 | Beispiel 14 |
| Aerosi® 200 | 12 | 7 | Aerosil® 200 |
| Ethanol 0,3k | 12 | 14 | Ethanol 03,kg |
| g | 12 | 15 | |
| 1,2 Diacyl-glycerol-3- | 14 | 18 | 1,2 Diacyl-glycero-3- |
| 1,2 Diacyl-glycerol-3- | 14 | 19 | 1,2 Diacyl-glycero-3- |
| 1,2 Diacyl-glycerol-3- | 14 | 20 | 1,2 Diacyl-glycero-3- |
| 1,2 Diacyl-glycerol-3- | 14 | 21 | 1,2 Diacyl-glycero-3- |
| 5-85% en poids d'acide | 15 | 47 | 75-85% en poids d'acide |
| rapporté à la la quantité totale | 15 | 51 | rapporté à la quantité totale |
| on séche le produit | 15 | 62 | on sèche le produit |

Tag der Entscheidung über die Berichtigung / Date of decision on rectification: / Date de décision portant sur modification: 17.10.89

Ausgabe- und Veröffentlichungstag: / Issue and publication date: / Date d'edition et de publication: 06.12.89

Patbl.Nr / EPB no: / Bull. no: 89/49

(19) Europäisches Patentamt
European Patent Office
Office européen des brevets

(11) Veröffentlichungsnummer: **0 147 741**
**B1**

(12) # EUROPÄISCHE PATENTSCHRIFT

(45) Veröffentlichungstag der Patentschrift: **02.08.89**

(51) Int. Cl.[4]: **A 61 K 31/685,** C 07 F 9/10

(21) Anmeldenummer: **84115401.6**

(22) Anmeldetag: **13.12.84**

(54) **Pharmazeutische Zubereitung mit speziellen 1,2-Diacyl-Glycero-3-Phosphocholinen zur Behandlung von Erkrankungen im Magen-Darmbereich.**

(30) Priorität: **22.12.83 DE 3346525**

(43) Veröffentlichungstag der Anmeldung:
**10.07.85 Patentblatt 85/28**

(45) Bekanntmachung des Hinweises auf die Patenterteilung:
**02.08.89 Patentblatt 89/31**

(84) Benannte Vertragsstaaten:
**AT BE CH DE FR GB IT LI LU NL SE**

(56) Entgegenhaltungen:
**EP-A-0 054 769**
**EP-A-0 092 121**
**DE-A-2 907 778**
**FR-A-2 343 481**

(73) Patentinhaber: **A. Nattermann & Cie. GmbH, Nattermannallee 1, D-5000 Köln 30 (DE)**

(72) Erfinder: **Schulz, Volker, Dr., Laudahnstrasse 37, D-5000 Köln 41 (DE)**
Erfinder: **Leyck, Sigurd, Dr., Am Quechenhauf 21, D-5024 Pulheim 2 (DE)**
Erfinder: **Dürr, Manfred, Dr., Am Blauen Stein 10, D-5024 Pulheim- Dansweiler (DE)**
Erfinder: **Ghyczy, Miklos, Dr., Am Serviesberg, D-5000 Köln 41 (DE)**
Erfinder: **Wendel, Armin, Max- Ernst- Strasse 20, D-5000 Köln 40 (DE)**
Erfinder: **Hager, Jörg, Hermann- Josef- Schmitt-Strassse 48, D-5000 Köln 30 (DE)**

(74) Vertreter: **Sternagel, Hans- Günther, Dr., Patentanwälte Dr. Michael Hann Dr. H.- G. Sternagel Sander Aue 30, D-5060 Bergisch Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 147 741 B1

LIBER, STOCKHOLM 1989

**Beschreibung**

Gegenstand der vorliegenden Erfindung ist die Verwendung von speziellem 1,2-Diacyl-glycero-3-phosphocholin, ggfs. im Gemisch mit anderen 1,2-Diacyl-glycero-3-phosphaten, mit 75 bis 86 Gew.-% ungesättigten Fettsäureresten mit einer Kettenlänge von $C_{16}$, $C_{18}$ und/oder $C_{20}$ zur Herstellung von pharmazeutischen Zubereitungen zur Behandlung von Erkrankungen im Magen-Darm-Bereich.

Aus DE-A-2 907 778 ist die Verwendung von Phosphatidylcholinderivaten und Phosphatidyläthanolaminderivaten in Arzneimitteln zur Bekämpfung von Wahrnehmungs- und Bewußtseinsstörungen bekannt.

Etwa 10 % bis 20 % der Menschen erkranken einmal oder mehrmals in ihrem Leben an einem peptischen Geschwür des Magens, des Zwölffingerdarmes bzw. an anderen entzündlichen Veränderungen des Magen-Darmbereiches. Die Ursachen sind vielfältig, z. B. darunter endogene zytotoxische Substanzen, wie Magen- und Gallensäuren, oder exogene Noxen, wie Ethanol oder Arzneimittel. So gehört der Gastrointestinaltrakt zu den am häufigsten von Arzneimittelnebenwirkungen betroffenen Organsystemen. Insbesondere führen nichtsteroidale Antiphlogistica zu Erosionen und Ulzerationen des Magens.

Die Therapie von Mukosaschäden (Ulcera) des Magens und des Zwölffingerdarmes beruht heute in erster Linie auf der Beseitigung von aggressiven Faktoren, wie der

- Ausschaltung von Säure und Pepsin durch Säureneutralisation (Antacida, wie z. B. Aluminiumhydroxide, Calciumcarbonat, Hydrotalcit, Magnesiumhydroxid, Natriumbicarbonat u.a.), Sekretionshemmung (Anticholinergika, $H_2$-Antagonisten, wie z. B. Cimetidin oder Ranitidin, Vagotomie oder distale Magenresektion) oder beschleunigte Elimination (Metoclopramid, Sulpirid);
- Verhütung einer zytotoxischen Wirkung bestimmter Bestandteile (Gallensäuren, Lysolecithin) des Duodenalsaftes im Magen durch Absorption (Cholestyramin, Antacida) oder beschleunigte Elimination (Metoclopramid, Sulpirid).

Daneben werden Filmbildner, wie sulfatierte Disaccharide (Sucralfate), angewendet.

Die bisherige Therapie geht also in erster Linie von einer Reduktion der Aktivität von Magensäure und Pepsin aus. Damit soll die "Durchbrechung" der Mukosabarriere verhindert werden. Diese Therapie hat jedoch zur Konsequenz, daß auch die Nahrungsverdauung beeinträchtigt wird, woraus wiederum weitere Störungen und Belastungen des Gastrointestinaltraktes resultieren.

Eine Reihe weiterer Nachteile dieser heute eingesetzten Therapeutika sind bekannt. So hat der saure Magensaft selbst auch Schutzfunktionen, z. B. bei der Bakterienabwehr. Diese natürliche Protektion geht verloren. Auch können Antacida je nach Zusammensetzung obstipierend, steinbildend oder resorptionshemmend für Mineralien und Pharmaka wirken. Ähnliche Auswirkungen und darüberhinaus weitergehende Störungen bewirken Anticholinergika und die neuerdings sehr häufig angewendeten $H_2$-Antagonisten. Es handelt sich um resorbierbare und daher systemisch im ganzen Organismus wirksame Arzneistoffe, die mit einer Vielzahl von Nebenwirkungen behaftet sind. Anticholinergika bewirken z. B. Mundtrockenheit und Akkomodationsstörungen; $H_2$-Antagonisten verursachen Kopfschmerzen, Diarrhoe, Gelenk- und Muskelschmerzen, Müdigkeit, Schwindel, Haarausfall, Störung im Sexualverhalten, Leberschäden u.a.

In EP-A-092 121 wurde auch vorgeschlagen, die Magenschleimhaut mit einem amphoteren Phospholipid-Surfactant zu überziehen, um Schädigungen zu vermeiden. Diese Überlegungen basieren auf der Schutzwirkung natürlicher Surfactanten in der Lunge. Die Beschaffenheit solcher 'Surfactanten' ist ausführlich untersucht, z. B.: B.H. Hills et al., J. Appl. Physiol.: Environ Exercise Physiol. 1982, 53 (1), 119 - 123; 1982, 53 (2), 463 - 469; 19B3, 54 (2), 420 - 426; Respiration Physiology 1983, 51, 79 - 93; L.M. Lichtenberger, Science 219 (1983), 1327 - 1329.

Als wichtigste Komponenten wurden identifiziert:
Dipalmitoylphosphatidylcholin (DPPC),
Dipalmitoylphosphatidylglycerol (DPPG),
Dipalmitoylphosphatidylethanolamin (DPPE) und
Sphingomyelin (SP).
Besonders vorteilhaft sind Kombinationen wie z. B.
DPPC : DPPE : DPPG : SP (5 : 2 : 2 : 1)
DPPC : DPPE : DPPG (5 : 2 : 2)
DPPC : DPPE (1 : 1)
DPPC : DPPG (1 : 1) oder
DPPC : DPPE : SP.

Um jedoch eine Filmbildung zu ermöglichen, ist es wichtig (B.A. Hills et al., A. J. Physiol. 244 (Gastrointest. Liver Physiol 7), G561 - G568, 1983), daß die Fettsäurereste in den Phospholipiden sich gerade ausrichten können. Es kommen daher nur gesättigte Fettsäurereste, insbesondere Palmitinsäure in Frage.

Die Anwendung solcher 'Surfactanten' zur Therapie von Mukosaschäden zeigt jedoch eine Reihe von Problemen:

- Die Gewinnung solcher natürlicher, komplex aufgebauter Surfactanten ist sehr aufwendig.
- Beim therapeutischen Einsatz solcher Surfactanten sind die überwiegend gesättigten Fettsäurereste im Phospholipid wegen ihres ungünstigen Einflusses auf den Fettstoffwechsel und der daraus resultierenden

Atherosklerosegefahr (M. Rosseneu et al., Atherosclerosis 32 (1979) 141 - 153) als äußerst problematisch anzusehen.

- Die Bildung eines Schutzfilms im Magen ist sehr problematisch. Die Übertragung der Funktion der Surfactants in den Lungenalveolen auf die Mucosa des Magen-Darmtraktes ist aus anatomischen und physiologischen Unterschieden der beiden Organsysteme nicht möglich.

Die Lungenalveolen sind von einem nichtsezernierendem einschichtigem flachem Endothel ausgekleidet, das eine gute Grundlage für einen stabilen molekularen Lipidfilm bildet. In funktioneller Hinsicht findet dort lediglich der Austausch kleiner Gasmoleküle ($O_2$, $CO_2$) statt, die problemlos durch den Lipidfilm hindurchdiffundieren.

Im Magen-Darm-Bereich finden dagegen intensive Sekretions- und Resorptionsvorgänge statt. Die letzteren betreffen insbesondere auch große wasserlösliche Moleküle, so daß eine Filmbildung kaum möglich ist.

Die gegenwärtige Ulkus-Therapie stellt eine symptomatische, aber keineswegs eine kurative Therapie dar. Sie heilt allenfalls das Ulkus, nicht aber die Ulkus-Krankheit.

Als alternatives therapeutisches Ziel wäre deshalb eine die protektiven Eigenschaften der Mukosa steigernde Therapie anzustreben. Mit Carbenoxolon wurde erstmals ein die protektiven Eigenschaften der Mukosa stimulierender Wirkstoff bekannt. Aufgrund von ausgeprägten Nebenwirkungen (Natrium- und Wasserretention, Hypokaliämie) ist die Anwendung jedoch nur begrenzt möglich.

Die experimentell gut dokumentierten Eigenschaften der Prostaglandine, wie Steigerung der Bicarbonat- und Mukussekretion, der Mukosadurchblutung und der Förderung der Epithelzellausreifung sowie der Magenentleerung weisen darauf hin, daß durch Gabe dieser Wirkstoffgruppe das therapeutische Prinzip einer Zytoprotektion in idealer Weise zu erfüllen wäre.

Die bisher klinisch geprüften langlebigen $PGE_2$-Analoga waren jedoch mit dem Nachteil erheblicher systemischer Nebenwirkungen behaftet.

Die natürlichen Prostaglandine wirken da gegen, wie in anderen Organen, auch im Verdauungstrakt als sogenannte lokale Hormone. Das heißt, daß sie nicht über den Blutstrom an den Ort ihrer Wirkung gelangen, sondern in unmittelbarer Nachbarschaft oder sogar direkt in den Geweben gebildet werden, in denen sie ihre kurzlebige Wirkung entfalten.

Erstrebenswert ist deshalb eine therapeutische Möglichkeit der Behandlung, die die physiologische Synthese der mukosaprotektiven Prostaglandine lokal in der Darmschleimhaut begünstigt. Dafür sind aus theoretischen Erwägungen insbesondere solche Substrate der Prostaglandinsynthese geeignet, die sich bei oraler Zufuhr schnell und dauerhaft in der Magen-Darm-Mukosa anreichern.

Aufgabe der vorliegenden Erfindung ist es, pharmazeutische Zubereitungen zur Behandlung von Erkrankungen im Magen-Darmbereich bereitzustellen, die sich bei oraler Zufuhr schnell und dauerhaft in der Magen-Darm-Mukosa anreichern und so als Substrat für die Synthese mukosaprotektiver Prostaglandine dienen können.

Die Aufgabe wird gelöst durch die Verwendung von 1,2-Diacylglycero-3-phosphocholin in dem
75 bis 36 Gew.-% der Acylreste ungesättigte Fettsäurereste sind und ein Gemisch aus Fettsäureresten aus
10 bis 20 Gew.-% Palmitinsäure
4 bis 5 Gew.-% Stearinsäure
10 bis 12 Gew.-% Ölsäure
62 bis 68 Gew.-% Linolsäure
3 bis 6 Gew.-% Linolensäure
sind, zur Herstellung einer pharmazeutischen Zubereitung zur Behandlung von Erkrankungen im Magen-Darm-Bereich.

Es wurde überraschend gefunden, daß spezielle 1,2-Diacylglycero-3-phosphocholine, worin Acyl für überwiegend ungesättigte Fettsäurereste mit 16 und/oder 18 und/oder 20 Kohlenstoffatomen steht, sich hervorragend zur Behandlung von Erkrankungen des Magen- Darmbereiches eignen. Die geeigneten speziellen 1,2-Diacyl-glycero-3-phosphocholine weisen 75 - 86 % ungesättigte Fettsäurereste auf.

Ganz besonders bevorzugt sind die 1,2-Diacyl-glycero-3-phosphocholine, bei denen der 1- und 2-Acylrest aus unterschiedlichen Gemischen von Fettsäurresten besteht.

Bei diesen bevorzugten 1,2-Diacyl-glycero-3-phosphocholinen besteht der Acylrest in 1-Stellung aus folgenden Fettsäurerest-Gemischen

22 - 26 Gew.-% Palmitinsäure
6 - 9 Gew.-% Stearinsäure
8 - 12 Gew.-% Ölsäure
50 - 54 Gew.-% Linolsäure
4 - 6 Gew.-% Linolensäure

und der Acylrest in 2-Stellung besteht aus folgenden Fettsäurerest-Gemischen

1 - 2 Gew.-% Palmitinsäure
0 - 1 Gew.-% Stearinsäure
8 - 12 Gew.-% Ölsäure

75 - 85 Gew.-% Linolsäure
5 - 8 Gew.-% Linolensäure,

wobei die einzelnen Gehalte so ausgewählt sind, daß sie jeweils 100 % ergeben.

Die 1,2-Diacyl-glycero-3-phosphocholine entsprechen der allgemeinen Formel

```
                         O
                         ||
R1OCH2CH(OR2)CH2OPOCH2CH2N+(CH3)3
                         |
                         O-
```

worin $R^1$ und $R^2$ gleich oder verschieden sein können und die Reste

$CH_3(CH_2)_1(CH{:}CHCH_2)_m(CH_2)_nCO-$

worin

1 = 1, 4, 7, 8 oder 10
m = 0, 1, 2, 3
n = 6

bedeuten, wie z. B. die folgenden Reste

$CH_3(CH_2)_{14}CO-$
$CH_3(CH_2)_{16}CO-$
$CH_3(CH_2)_7CH{:}CHCH_2(CH_2)_6CO-$
$CH_3(CH_2)_4(CH{:}CHCH_2)_2(CH_2)_6CO-$
$CH_3CH_2(CH{:}CHCH_2)_3(CH_2)_6CO-$

Die pharmazeutischen Zubereitungen mit speziellen 1,2-Diacyl-glycero-3-phosphocholine können ggfs. bis zu 20 % weitere 1,2-Diacyl-glycero-3-phosphate bzw. deren Gemische, wie z. B. 1,2-Diacyl-glycero-3-phosphoethanolamin, 1,2-Diacyl-glycero-3-phosphoinositol, 1,2-Diacyl-glycero-3-phosphoserin, 1,2-Diacyl-glycero-3-phosphoglycerol, insbesondere jedoch 1,2-Diacyl-glycero-3-phosphoethanolamin enthalten,

wobei die Acylreste die gleiche Zusammensetzung haben wie für die 1,2-Diacyl-glycero-3-phosphocholine angegeben,

Diese 1,2-Diacylglycero-3-phosphocholine können nach an sich bekannten Verfahren (EP-A-54 770, EP-A-54 768, EP-A-54 769), insbesondere aus Sojabohnen, gewonnen werden.

Die speziellen 1,2-Diacyl-glycero-3-phosphocholine werden, wie Ganztier-Autoradiographien zeigen, nach oraler Zufuhr schnell und dauerhaft in der Magen- und Darmschleimhaut angereichert. In Tierexperimenten wurde nachgewiesen, daß die Vorbehandlung mit speziellen 1,2-Diacyl-glycero-3-phosphocholinen die Versuchstiere vor Magenschleimhautschäden, ausgelöst durch schleimhautreizende Agenzien, eindrucksvoll schützt.

Klinische Untersuchungen wurden bei 9 Patienten durchgeführt. Dabei wurden 7 charakteristische, subjektive Parameter (Sodbrennen, Schmerzen im Epigastrium, Völlegefühl, Appetitlosigkeit, Brechreiz, Übelkeit, Singultus), die im Zusammenhang mit Schleimhauterosionen und -ulcerationen im Magen-Duodenal-Bereich auftreten, vor und nach Behandlung mit 1,2-Diacyl-glycero-3-phosphocholin erfaßt. Die Dokumentation erfolgte halbquantitativ unter Berücksichtigung der Intensität der Beschwerden. Bei 8 der 9 Patienten (darunter 4 unter der Einnahme von nichtsteroidalen Antirheumatika, 1 unter Zytostatika, 1 mit Magenstumpf-Gastritis, 1 mit galligem Reflux, 1 mit chronisch-rezidivierender Ulcuskrankheit) wurden innerhalb von 1 - 4 Behandlungstagen wesentliche, zum Teil dramatische Besserungen der Beschwerden registriert. Nebenwirkungen oder Beeinträchtigung der sonstigen medikamentösen Therapie traten nicht auf. Wie die tierexperimentellen und klinischen Untersuchungen zeigen, führt die orale sowie die parenterale Behandlung mit speziellem 1,2-Diacyl-glycero-3-phosphocholin zu einem nachhaltigen Schutz der Magenschleimhaut. Die zytoprotektive Wirkung läßt sich als eine lokale Stimulation der Prostaglandinsynthese aufgrund einer Anreicherung des 1,2-Diacyl-glycero-3-phosphocholins in der Schleimhaut erklären. Systemische, vermehrte Prostaglandinwirkungen, wie sie bei der Prüfung langlebiger $PGE_2$-Analoga beobachtet wurden, konnten nicht festgestellt werden.

Wie die Untersuchungen zeigen, führt die orale sowie die parenterale Verabreichung der speziellen 1,2-Diacyl-glycero-3-phosphocholine zu einem nachhaltigen Schutz der Magenschleimhaut. Dies ist besonders überraschend und war nach Kenntnis der EP-A-092 121 nicht zu erwarten, da die hochungesättigten 1,2-Diacyl-glycero-3-phosphocholine bei den physiologischen Bedingungen nicht in der Lage sind, einen brauchbaren Schutzfilm zu bilden. Darüber hinaus war schon gar nicht zu erwarten, daß auch die parenterale Verabreichung zu einem nachhaltigen Schutz der Magenschleimhaut führt, da hier eine Filmbildung völlig ausgeschlossen ist.

Die erfindungsgemäß zu verwendenden speziellen 1,2-Diacyl-glycero-3-phosphocholine können oral sowie

parenteral verabreicht werden in Dosierungen von 0,2 bis 150 mg, insbesondere 10 - 50 mg pro kg Körpergewicht, ein- oder mehrmals täglich. Sie können prophylaktisch oder therapeutisch verabreicht werden. Weiterhin ist eine Verabreichung bei der Therapie mit Arzneimitteln, die Erosionen und Ulzerationen des Magens verursachen, möglich. Zur Verhinderung bzw. zur Beseitigung von Magenschleimhautschäden, die durch Arzneimittel verursacht werden, können die 1,2-Diacyl-glycero-3-phosphocholine vor, während oder nach der Therapie mit diesen Arzneimitteln verabreicht werden. Bevorzugt erfolgt eine Vorbehandlung durch Verabreichung von 1,2-Diacyl-glycero-3-phosphocholin oder durch gleichzeitige Gabe von Arzneimitteln, um mögliche Magenschleimhautschäden durch das Arzneimittel zu vermeiden.

Bei der Behandlung von schweren Ulzerationen kann man zur Verminderung oder Beseitigung der aggressiven Faktoren Ulcustherapeutica, wie z. B. $H_2$-Antagonisten, einsetzen und dann zu einer Therapie mit 1,2-Diacyl-glycero-3-phosphocholinen übergehen. Dies hat den Vorteil, daß die Nebenwirkungen der Ulcustherapeutica verringert werden.

Insbesondere bei der Behandlung von Krankheiten mit Magen-Darm-aggressiven Wirkstoffen, z. B. mit Zytostatica, wie Methohexat; Antihyertonica, wie Reserpin; Tuberkulostatica, wie Rifampicin; oder p-Aminosalicylsäure; Antibiotica, wie Penicillin; oder Analgetica bzw. Antiphlogistica, wie Pyrazolone (Phenylbutazon, Oxyphenbutazon), Salicylsäurederivate (Salicylsäure, Salicylsäureamid, Acetylsalicylsäure, Benorilat, Diflunisal); Phenylalkansäuren, wie z. B. Ibuprofen, Naproxen, Alclofenac, Ketoprofen, Diclofenac, Fenoprofen, Tolmetin, Flurbiprofen, Suprofen, Indoprofen, Carprofen, Pirprofen, Fenclofenac, Sulindac, Indometacin, Piroxicam, Acemetacin, Pimetacin, Sulindac, Nambumeton u.a., ist es vorteilhaft, eine Vorbehandlung mit und/oder gleichzeitiger Verabreichung in einer Dosierung oder getrennten Dosierungen mit dem speziellen 1,2-Diacyl-glycero-3-phosphocholin vorzunehmen.

Zur Verabreichung der speziellen 1,2-Diacyl-glycero-3-phosphocholine werden diese in eine geeignete Form überführt wie z. B. Kapseln, Lösungen, Emulsionen, Tabletten, Pulver, Kaukapseln, Trinkampullen oder wässrige Emulsionen sowie in übliche parenterale Formen.

Die speziellen 1,2-Diacyl-glycero-3-phosphocholine können nach an sich bekannten Verfahren in Weichgelatinekapseln oder vorteilhafter in Hartgelatinekapseln, z. B. nach dem in der DE-A-3 022 136 beschriebenen Verfahren, ggfs. unter Zusatz geeigneter Hilfs- und Füllstoffe, abgefüllt werden. Die Weich- oder Hartgelatinekapseln können als Ganzes geschluckt oder auch gekaut werden.

Zur Herstellung der Hartgelatinekaukapseln werden die Phospholipide mit pharmazeutisch inerten Trägerstoffen, wie z. B. Wachse, hydrierte Öle, natürliche, halbsynthetische oder synthetische Triglyceride und deren Gemische, wie Kakaobutter, sowie übliche Suppositorienmassen, z. B. auf Triglyceridbasis, wie z. B. Witepsol-Suppositorienmassen (vergl. H.P. Fiedler, Lexikon der Hilfsstoffe für Pharmazie, Kosmetik und angrenzende Gebiete, 1971, Bd. 9, S. 548 - 50 und 632 - 634); Fettalkohole; feste Kohlenwasserstoffe, wie Vaseline oder Paraffin solidum; gesättigte Fettsäuren, wie Laurinsäure, Myristinsäure, Palmitinsäure, Stearinsäure; Emulgatoren, wie ethoxylierte Triglyceride, polyethoxylierte pflanzliche Öle; Fettsäurezuckerester; Silikone; Gelatine; Methylcellulose; Hydroxypropoxycellulose; Hydroxypropylcellulose; Polyethylenglykole; Polyvinylpyrrolidon; Polyvinylalkohol; Polyacrylsäure und deren Salze gemischt. Den Massen wird Ethanol in einer solchen Menge zugesetzt, daß ein bei Raumtemperatur oder schwach erhöhter Temperatur unter Druck, fließfähiges Füllgut erhalten wird, d. h., daß das Produkt bei dieser Temperatur eine zum Transport unter Druck gerade genügend geringe oder wenig geringere Viskosität hat, und dieses Produkt auf den bekannten Abfüllanlagen mit Zusatzeinrichtung zur Flüssigabfüllung und mit erwärmbarer Abfülldüse analog der in der DE-A-3 022 136 beschriebenen Methode abgefüllt werden kann.

Die 1,2-Diacyl-glycero-3-phosphocholine können auch zusammen mit anderen Ulcustherapeutica oder auch mit Arzneimitteln, die Mukosa-schädigende Nebenwirkungen zeigen, nach dem Verfahren verarbeitet werden, indem diese z. B. in die viskosen 1,2-Diacyl-glycero-3-phosphochlin-Massen vor der Abfüllung eingearbeitet werden, oder nach Abfüllung der 1,2-Diacylglycero-3-phosphocholine in Pulver- oder Tablettenform zudosiert werden, wobei das Verhältnis von 1,2-Diacyl-glycero-3-phosphocholin und weiteren Wirkstoffen von 0,1 : 1 bis 1 : 20 variieren kann.

Zur Herstellung von Tabletten und Pulvermischungen müssen die 1,2-Diacyl-glycero-3-phosphocholine in eine feste Form überführt werden, was wegen der viskosen Eigenschaften dieser Substanzen äußerst schwierig ist. Durch Zusatz von 2 - 10 Gew.-% Calziumchlorid können feste Zubereitungen erhalten werden.

Zur Herstellung der festen Zubereitungen werden die speziellen 1,2-Diacyl-glycero-3-phosphocholine unter Zusatz üblicher Hilfsstoffe in Wasser oder einem organischen Lösungsmittel, wie z. B. Alkohole, wie Methanol, Ethanol oder Isopropanol, in Kohlenwasserstoffen, wie Hexan, chloriden Kohlenwasserstoffen oder Gemischen davon, gelöst bzw. emulgiert, das Calciumchlorid zugegeben und unter leichter Erwärmung gerührt und anschließend das Lösungsmittel wieder abgezogen, wobei ein trockenes Pulver erhalten wird. Das Calziumchlorid wird dabei in Mengen von 1 - 20 %, insbesondere jedoch 2 - 10 %, bezogen auf die Gewichtsmenge 1,2-Diacyl-glycero-3-phosphocholins zugesetzt.

Bevorzugte Lösungsmittel für die Verarbeitung von 1,2-Diacyl-glycero-3-phosphocholinen sind Alkohole, insbesondere Ethanol. Schlämmittel sind Wasser oder Alkohole, wie Methanol oder Ethanol.

Zur Trocknung eignen sich übliche Verfahren, wie Vakuum-Walzen-Trocknung, Sprühtrocknung, Gefriertrocknung. Die getrockneten, zerkleinerten 1,2-Diacylglycero-3-phosphocholin-Calziumchlorid-Mischungen können gegebenenfalls nach üblichen Verfahren zerkleinert bzw. granuliert werden. Zur Stabilisierung der Produkte werden vorteilhafterweise 0,1 - 2 Gew.-%, bezogen auf die eingesetzte 1,2-Diacylglycero-3-phosphocholin-Menge, eines Stabilisators oder Stabilisatorgemisches, wie Toxopherolacetat und/oder

Ascorbinsäurepalmit, eingesetzt.

Die speziellen 1,2-Diacyl-glycero-3-phosphocholine können auch mit Arzneimitteln, die mukosaschädigende Nebenwirkungen zeigen, zusammen verarbeitet werden. Zu den Arzneimitteln zählen z. B.: Zytostatica, Chemotherapeutica, Antibiotica, Steroide, insbesondere steroidale und nichtsteroidale Antiphlogistica.

Die Herstellung der festen, oralen Arzneiformen, bestehend aus Arzneistoff bzw. nichtsteroidalen Antiphlogistica und speziellem 1,2-Diacyl-glycero-3-phosphocholin, kann nach verschiedenen Verfahren erfolgen.:

Pulvermischungen:

Die auf entsprechende Korngröße zerkleinerten Arzneistoffe und ein spezielles 1,2-Diacyl-glycero-3-phosphocholin-Calziumchlorid-Gemisch werden unter Zusatz üblicher galenischer Hilfsstoffe gemischt und zu Tabletten gepreßt oder in Kapseln abgefüllt.

Sprüheinbettungen:

Die Lösung bzw. Dispersion des Arzneistoffes in organischen Lösungsmitteln oder Wasser werden mit einer Lösung oder Emulsion des speziellen 1,2-Diacyl-glycero-3-phosphocholins in organischen Lösungsmitteln bzw. Wasser und der Lösung des Calziumchlorids in organischen Lösungsmitteln bzw. Wasser, sowie ggfs. weiteren üblichen galenischen Hilfsstoffen gemischt und sprühgetrocknet. Die erhaltene Sprüheinbettung kann unter Zusatz weiterer galenischer Hilfsstoffe entweder zu Tabletten gepreßt oder in Kapseln abgefüllt werden.

Umhüllungen:

Die Lösung oder Emulsion des speziellen 1,2-Diacyl-glycero-3-phosphocholins in organischen Lösungsmitteln bzw. Wasser wird mit einer Lösung des Calziumchlorids in organischen Lösungsmitteln bzw. Wasser gemischt und im Fließbett auf den vorher auf die gewünschte Korngröße zerkleinerten Arzneistoff aufgetragen. Das erhaltene pulverförmige, freifließende Produkt wird unter Zusatz von galenischen Hilfsstoffen entweder zu Tabletten gepreßt oder in Kapseln abgefüllt.

Das Verhältnis Arzneistoff zu 1,2-Diacyl-glycero-3-phosphocholin/Calziumchlorid kann je nach therapeutischen Erfordernissen im Gewichtsverhältnis von 1 : 0,1 bis 1 : 20 variieren. Vorteilhafterweise werden je Dosierung des Arzneistoffes 50 - 250 mg, insbesondere 100 mg, des 1,2-Diacyl-Calziumchlorid-Gemisches eingesetzt.

Der Vorteil der festen, oralen Verabreichungsformen, bestehend aus Arzneistoffen und speziellem 1,2-Diacyl-glycero-3-phosphocholin-Calziumchlorid-Gemisch, gegenüber Formulierungen, bestehend aus Arzneistoffen und Phospholipiden, wie sie z. B. in DE-B-2 856 333 beschrieben sind, kann wie folgend bestimmt werden: Es wird der Anteil an emulgiertem Phospholipid bzw. 1,2-Diacyl-glycero-3-phosphocholin aus einer festen, oralen Arzneiform, bestehend aus Arzneistoff und Phospholipid bzw. Arzneistoff und 1,2-Diacyl-glycero-3-phosphocholin/Calziumchlorid, im Magensaft bestimmt nach dem USP-Dissolution Test (Drehkörbchen-Methode, 100 UPM, 900 ml Magensaft, pH 1,2), Material:

| | |
|---|---|
| AS = | Phenylbutazon |
| PL = | Phosphatidylcholin |
| $PC/CaCl_2$ = | 1,2-Diacyl-glycero-3-phosphocholin/Calziumchlorid (Gehalt: 6,43 % $CaCl_3$) |

Kapseln zu je 200 mg AS und 100 mg PL bzw. $PC/CaCl_2$

| Produkt | emulgierte/dispergierte Menge nach 30 Minuten |
|---|---|
| 1. PL + AS | 37,7 mg |
| 2. $PC/CaCl_2$ + AS | 67,5 mg |

Als weiterer Vorteil der bevorzugten Zubereitungen, bestehend aus Arzneistoff und 1,2-Diacyl-glycero-3-phosphocholin/Calziumchlorid, gegenüber Arzneiformen, bestehend aus Arzneistoff und Phospholipid, ist die Möglichkeit zu verzeichnen, daß nach dem Verfahren hohe und erforderliche Mengen an 1,2-Diacyl-glycero-3-phosphocholin in Form von Mischungen mit $CaCl_2$ und Arzneistoff kombiniert werden können. Z. B. können nach dem bekannten Fließbettverfahren Acetylsalicylsäure mit Phosphatidylcholin nur maximale Mischungsverhältnisse von 1 : 0,2 erhalten werden, wogegen Acetylsalicylsäuren mit dem speziellen 1,2-Diacyl-glycero-3-phosphocholin/Calziumchlorid sich zu Gemischen von über 1 : 0,5 herstellen lassen.

Ein weiterer großer Vorteil sind die pulvertechnologischen Eigenschaften der mit dem speziellen 1,2-Diacyl-glycero-3-phosphocholin/Calziumchlorid gefertigten pharmazeutischen Zubereitungen.

| Produkt | Schüttgewicht nach DIN 53912 | Rieselfähigkeit nach DIN 53916 |
|---|---|---|
| 1. ASS + PL | 227 g/l | nicht rieselfähig |
| 2. ASS + $PC/CaCl_2$ | 257 g/l | $\cot \varphi = 1{,}43$ |

ASS = Acetylsalicylsäure
PL = Phosphatidylcholin
PC = 1,2-Diacylglycero-3-phosphocholin

Der Vorteil der pulvertechnologischen Eigenschaften der mit Mischungen 1,2-Diacyl-glycero-3-phosphocholin/Calziumchlorid gefertigen Arzneiformen ist den vorteilhaften physikalischen Eigenschaften der 1,2-Diacyl-glycero-3-phosphocholin-Calziumchlorid-Mischung gegenüber dem reinen 1,2-Diacyl-glycero-3-phosphocholin zuzuschreiben.

| Produktbezeichnung | Schüttgewicht nach DIN 53912 |
|---|---|
| 1. PC1 - gemahlen | 229 g/l |
| 2. PC1/$CaCl_2$ - gemahlen | 451 g/l |
| 3. PC1 - sprühgetrocknet | 88 g/l |
| 4. PC1/$CaCl_2$ - strühgetrocknet | 222 g/l |
| 5. PC2 - gemahlen | 230 g/l |
| 6. PC2/$CaCl_2$ - gemahlen | 440 g/l |

PC1 = 1,2-Diacyl-glycero-3-phosphocholin
PC2 = Gemisch aus
80 % 1,2-Diacyl-glycero-3-phosphocholin
20 % 1,2-Diacyl-glycero-3-phosphoethanolamin

Sollen die speziellen 1,2-Diacyl-glycero-3-phosphocholine zusammen mit anderen Arzneimitteln verabreicht werden, können auch Arzneimittelkerne mit dem 1,2-Diacyl-glycero-3-phosphocholin überzogen werden. Auch sind Kombinationspackungen möglich, die das zu verabreichende Arzneimittel und die Zubereitung aus speziellem 1,2-Diacyl-glycero-3-phosphocholin enthalten.

**Beispiel 1**

Zum Nachweis der Wirksamkeit wurden folgende Versuche durchgeführt:
Anreicherung von speziellem 1,2-Diacyl-glycero-3-phosphocholin in der Magen-Darm-Mukosa.
Ratten erhielten 70 mg/kg mit $^3H$ radioaktiv markiertem Phosphocholin (1,2-/9,10,12,13-$^3H_4$/-Dilinolyl-sn-glycero-3-phosphocholin) per Schlundsonde. Die Radioaktivität betrug pro Tier 4,4 x $10^6$ dpm $^3H$. Die Ratten wurden 1,5; 3; 6; 24 Stunden nach Applikation in tiefer Äthernarkose durch Tauchen in eine Aceton-Trockeneis-Mischung (-72°C) getötet. Es wurden Ganzkörperschnitte hergestellt. Die trockenen Schnitte wurden zusammen mit 2 Standards verschiedener Radioaktivität unter gleichmäßigem Druck in Kontakt mit der photographischen Emulsion gebracht und bei -20°C exponiert. Die Expositionsdauer betrug 47 Tage. Es konnte durch diese Autoradiographie-Abbildung gezeigt werden, daß das verabreichte 1,2-Diacyl-glycero- 3-phosphocholin schnell und langanhaltend zu einer Anreicherung in der Magen-Darm-Mukosa führt, was durch eine starke hervortretende Schwärzung der Magenwand auf der Abbildung zum Ausdruck kommt.

**Beispiel 2**

Chemische Schleimhautschädigung.
Eine ulzerogene Phenylbutazon-Dosis (200 mg/kg, 5 ml/kg) wurde an männlichen Ratten, die 3 Tage lang nur mit Weißbrot gefüttert wurden und danach 24 Stunden Nahrungskarenz hatten, oral verabreicht. Unmittelbar danach erfolgte die orale Applikation der Testsubstanz. Nach 3,5 Stunden wurden die Tiere durch Äthernarkose getötet und die Mägen makroskopisch untersucht. Die Verwertung der Ulzeration erfolgte nach Takagi und Okabe (Jap. J. Pharmacol. 18, 9 - 18 (1968) mit Hilfe einer Indexzahl.

| Testsubstanz 120 mg/kg | Ulcusindex Kontrolle | Ulcusindex Versuch | Änderung des Ulcusfaktors in % |
|---|---|---|---|
| PPC | 3,2 | 1,6 | - 50,0 % |
| DPPC | 1,9 | 1,2 | - 36,8 % |
| DPPC/DPPG 1 : 1 | 2,7 | 2,1 | - 22,2 % |
| DPPC/DPPE/ DPPG/DPPI/Sph. 9 : 1 : 1 : 1 : 1 | 2,5 | 2,0 | - 20,0 % |

| | |
|---|---|
| PPC = | 1,2-Diacyl-glycero-3-phosphocholin (Acylreste ca. 62 - 68 % Linolsäure) gem. Anspruch 3 |
| DPPC = | Dipalmitoylphosphatidylcholin |
| DPPG = | Dipalmotoylphosphatidylglycerol |
| OPPI = | Dipalmitoylphosphatidylethanolamin |
| Sph = | Sphingomyclin |

Die Mischung DPPC/DPPE/DPPG/DPPI/Sph (9 : 1 : 1 : 1 : 1) entspricht der wirksamsten Mischung aus EP-0 092 121.
Wie aus der Tabelle ersichtlich, führten die speziellen 1,2-Diacylglycero-3-phosphocholine eindeutig zur stärksten Hemmung der Ulcusprovokation.

**Beispiel 3**

1,2-Diacyl-glycero-3-phosphocholin-Emulsion.

| | |
|---|---|
| 1,2-Diacyl-glycero-3-phosphocholin | 45,0 g |
| Trockenglykose (Stärkehydrolysat) | 5,0 g |
| Aromastoffe | 1,0 g |
| Konservierungsstoffe | 0,1 g |
| Wasser ad. | 100,0 g |

Das 1,2-Diacyl-glycero-3-phosphocholin wird in dem mit den Konservierungsstoffen versetztem Wasser unter Rühren emulgiert und anschließend die Trockenglukose und Aromastoffe zugemischt. Die viskose Emulsion wird in Tuben abgefüllt.

**Beispiel 4**

Hartgelatinekapseln

a) Rezeptur und Herstellung des Füllgutes

| | |
|---|---|
| 1,2-Diacyl-glycero-3-phosphocholin | 76,0 kg |
| Hartfett | 13,5 kg |
| Sojaöl | 8,5 kg |
| Ethanol | 2,0 kg |
| Pfefferminzöl | 0,04 kg |

werden in einem Kneter homogen gemischt.

b) Abfüllung in Hartgelatinekapseln.
Die unter a) beschriebene Masse wird auf einer Hartgelatinekapsel-Maschine mit einer Abfüllstation für flüssige Füllgüter bei 60°C in Hartgelatinekapseln abgefüllt. Kapselgröße 0 elongated, Dosierung 658 mg.

**Beispiel 5**

Trinkampullen

| | |
|---|---|
| 1,2-Diacyl-glycero-3-phosphocholin | 97,8 g |
| Kombisirup | 160,0 g |
| Mokkaessenz | 2,0 ml |
| Marsalaessenz | 3,2 ml |
| gereinigtes Wasser ad | 2000,0 ml |

Das 1,2-Diacyl-glycero-3-phosphocholin wird in 1,4 l Wasser unter Rühren dispergiert und anschließend mit einem Spalthomogenisator homogenisiert. Nach Zusatz einer wässrigen Lösung des Kombisirups und der Aromen wird mit Wasser auf 2 l aufgefüllt und steril filtiert. Anschließend wird die Lösung unter aseptischen Bedingungen in Injektionsfläschchen (5 ml) abgefüllt.

**Beispiel 6**

98 g 1,2-Diacyl-glycero-3-phosphocholin werden in Ethanol unter Erwärmung gelöst und mit einer Lösung von 2 g Calziumchlorid (wasserfrei) in 50 ml Methanol gemischt. Die Lösungsmittel werden unter Vakuum entfernt und das erhaltene Produkt unter Zusatz von 1 % Aerosil® 200 auf eine Korngröße < 100 µm zerkleinert. Das erhaltene Pulver wird in Kapseln abgefüllt.

Das Gemisch kann auch unter Zusatz geeigneter Hilfsstoffe zu Tabletten gepreßt werden.

**Beispiel 7**

90 g 1,2-Diacyl-glycero-3-phosphocholin werden in 200 ml Chloroform gelöst und mit einer Lösung von 10 g $CaCl_2$ wasserfrei in 200 ml Methanol gemischt und mittels eines Sprühtrockners, Typ Büchi 190, getrocknet. Das erhaltene Produkt wird unter Zusatz von 1 % Aerosil® 200 auf eine Korngröße < 100 µm zerkleinert und kann dann in Kapseln abgefüllt oder zu Tabletten verpreßt werden.

**Beispiel 8**

94 g eines Gemisches aus 80 Gew.-% 1 2-Diacyl-glycero-3-phosphocholin und 20 Gew.-% 1,2-Diacyl-glycero-3-phosphoethanolamin werden in Ethanol gelöst und mit einer Lösung von 6 g $CaCl_2$ (wasserfrei) in 120 ml Ethanol gemischt. Die Lösungsmittel werden unter Vakuum entfernt und das erhaltene Produkt analog Beispiel 6 verarbeitet.

**Beispiel 9**

90 g 1,2-Diacyl-glycero-3-phosphocholin werden in 500 ml aqua dest. emulgiert und mit einer Lösung vom 10 g $CaCl_2$ wasserfrei in 50 ml aqua dest. gemischt und analog Beispiel 6 verarbeitet.

**Beispiel 10**

Wässrige Dispersion.

| | |
|---|---|
| 1,2 Diacyl-glycero-3-phosphocholin/$CaCl_2$ | 56,0 kg |
| Sahnearoma | 0,8 kg |
| Mokkaschokoladeessenz | 5,6 kg |
| Vanillin | 0,4 kg |
| Dextrose | 37,0 kg |
| Saccharin-Natrium | 0,16 kg |

werden in einem Pulvermischer homogen gemischt.

Zur Herstellung der trinkfertigen wässrigen Dispersion werden unmittelbar vor der Anwendung 12,5 g Pulvergemisch in 100 ml Wasser gegeben und kurz geschüttelt.

**Beispiel 11**

ASS-Tabletten:

| | |
|---|---|
| ASS = Acetylsalicylsäure | 300 mg |
| 1,2-Diacyl-glycero-3-phosphocholin-$CaCl_2$ | 100 mg |
| Aerosil® 200 | 20 mg |
| Na-Carboxymethylcellulose | 20 mg |
| Rizinusöl hydriert | 10 mg |
| Cellulose microcristallin | 150 mg |

Die aufgeführten Ingredienzien werden gemischt, gepreßt und in ähnlicher Weise mit bekannten Tablettenüberzugsmitteln, z. B. Celluloseacetatphthalat oder Hydroxypropylmethylcellulosephthalat versehen.

**Beispiel 12**

| | |
|---|---|
| ASS-Kapseln: | |
| ASS = Acetylsalicylsäure, gemahlen < 100 µm | 300 mg |
| 1,2-Diacyl-glycero-3-phosphocholin-$CaCl_2$ | 100 mg |
| Trägerstoff | 15 mg |

Eine Lösung, bestehend aus 90 g 1,2-Diacylglycero-3-phosphocholin/$CaCL_2$, 200 ml Chloroform und 10 g $CaCl_2$-wasserfrei sowie 200 ml Methanol, wird im Fließbett auf ASS, gemahlen < 100 µ, aufgesprüht. Das erhaltene, freifließende Pulver wird in Hartgelatinekapseln zu 300 mg ASS-Kapseln abgefüllt.

**Beispiel 13**

| | |
|---|---|
| Phenylbutazon-Tabletten: | |
| Phenylbutazon | 200 mg |
| 1,2-Diacyl-glycero-3-phosphocholin-$CaCl_2$ | 100 mg |
| Aerosil® 200 | 30 mg |
| Na-Carboxymethylcellulose | 20 mg |
| Rizinusöl hydriert | 10 mg |
| Cellulose microcristallin | 140 mg |

200 g Phenylbutazon werden in 500 ml Chloroform gelöst, 93,5 g Phospholipid eingerührt und 6,5 g $CaCl_2$, gelöst in 100 ml Methanol, hinzugefügt. Die erhaltene Mischung wird sprühgetrocknet, mit den restlichen Komponenten gemischt, zerkleinert, zu je 200 mg Phenylbutazon-Tabletten gepreßt und analog Beispiel 11 überzogen.

**Beispiel 14**

| | |
|---|---|
| Indometacin-Kapseln: | |
| Indometacin | 50 mg |
| 1,2-Diacyl-glycero-3-phosphocholin-$CaCl_2$ | 100 mg |
| Trägerstoff | 5 mg |

Eine Lösung, bestehend aus 90 g 1,2-Diacyl-glycero-3-phosphocholin in 500 ml Isopropanol sowie 10 g $CaCl_2$ in 100 ml Methanol, wird im Fließbett auf das gemahlene Indometacin aufgesprüht. Das erhaltene Pulver wird, wie in Beispiel 6 beschrieben, behandelt.

**Beispiel 15**

| | |
|---|---|
| Ibuprofen-Tabletten: | |
| Ibuprofen | 200 mg |
| 1,2-Diacyl-glycero-3-phosphocholin-$CaCl_2$ | 100 mg |
| Aerosil® 200 | 30 mg |
| Cellulosemicrocristallin | 150 mg |
| Natriumcarboxymethylstärke | 20 mg |
| Magnesiumstearat | 10 mg |

Herstellung analog Beispiel 6.

**Beispiel 16**

| | |
|---|---|
| Ibuprofen-Kapseln: | |
| Ibuprofen | 200 mg |

| | |
|---|---|
| 1,2-Diacyl-glycero-3-phosphocholin-$CaCl_2$ | 100 mg |
| Trägerstoff | 10 mg |

Herstellung analog Beispiel 12.

**Beispiel 17**

Sulindac-Tabletten:

| | |
|---|---|
| Sulindac | 100 mg |
| 1,2-Diacyl-glycero-3-phosphocholin-$CaCl_2$ | 100 mg |
| Aerosil® 200 | 20 mg |
| Cellulose microcristallin | 100 mg |
| Natriumcarboxymethylcellulose | 20 mg |
| Magnesiumstearat | 10 mg |

Herstellung analog Beispiel 11.

**Beispiel 18**

Naproxen-Kapseln:

| | |
|---|---|
| Naproxen | 250 mg |
| 1,2-Diacyl-glycero-3-phosphocholin-$CaCl_2$ | 100 mg |
| Adsorptionsmittel | 15 mg |

Herstellung analog Beispiel 12.

**Beispiel 19**

Diclofenac-Natrium-Tabletten:

| | |
|---|---|
| Diclofenac-Natrium | 50 mg |
| 1,2-Diacyl-glycero-3-phosphocholin | 100 mg |
| Aerosil® 200 | 15 mg |
| Cellulose microcristallin | 75 mg |
| Magnesiumstearat | 10 mg |
| Natrium-carboxymethylcellulose | 5 mg |

Herstellung analog Beispiel 11.

**Beispiel 20**

Diclofenac-haltige Tabletten:

| | |
|---|---|
| Diclofenac-Natrium | 1000 g |
| Avicel® PH 102 | 1460 g |
| Aerosil® 200 | 20 g |
| Magnesiumstearat | 25 g |
| Natrium-carboxymethylstärke | 25 g |

Die Mischung wird zu gewölbten Tabletten von je 150 mg Diclofenac-Natrium verpreßt und anschließend mit einer Überzugsmasse aus

| | |
|---|---|
| 1,2-Diacyl-glycero-3-phosphocholin | 15,0 % |
| Hydroxypropylmethylcellulose | 7,5 % |
| Koloides $SiO_2$ | 1,5 % |
| Ethanol | 38,0 % |
| Methylenchlorid | 38,0 % |

in Mengen von ca. 100 mg je Tablette beschichtet.

**Beispiel 21**

Diclofenac-haltige Kapseln:

| | | |
|---|---|---|
| a) | Diclofenac-Natrium | 1000 g |
| | Avicel® PH 102 | 460 g |
| | Aerosi® 200 | 10 g |
| | Magnesiumstearat | 15 g |
| | Natrium-carboxymethylstärke | 15 g |
| b) | Füllmasse: | |
| | 1,2-Diacyl-glycero-3-phosphocholin | 9 kg |
| | Polyethylenglykol 400 | 1 kg |
| | Ethanol | 0,3 kg |

Die Mischung wird in einem Kneter bei 60° C homogen gemischt.

c) von der unter b) beschriebenen Füllmasse werden auf einer Hartgelatinekapsel-Maschine mit Zusatzteil für Flüssigabfüllung je 350 mg der Mischung pro Hartgelatinekapsel der Größe 0 dosiert. Anschließend werden auf derselben Hartgelatinekapsel-Abfüllanlage mit Hilfe einer Abfüllstation für Pulver 150 mg der unter a) beschriebenen Pulvermischung pro Kapsel zudosiert und die Kapsel verschlossen.

Die Kapseln enthalten 50 bis 1500 mg 1,2-Diacyl-glycero-3-phosphocholin sowie 10 - 250 mg nichtsteroidale Antiphlogistica, wobei das Verhältnis von Antiphlogistica zu 1,2-Diacyl-glycero-3-phosphocholin vorteilhafterweise 1 : 01 bis 1 : 10 beträgt.

**Beispiel 22**

Diclofenac-haltige Kapseln:

| | | |
|---|---|---|
| a) | Diclofenac-Natrium | 1000 g |
| | Avicel® PH 102 | 460 g |
| | Aerosil® 200 | 10 g |
| | Magnesiumstearat | 15 g |
| | Natrium-carboxymethylstärke | 15 g |

Die Stoffe werden homogen gemischt und zu 150 mg Tabletten mit einem Durchmesser von 6,5 mm verpreßt.

| | | |
|---|---|---|
| b) | Füllmasse | |
| | 1,2-Diacyl-glycero-3-phosphocholin | 76,5 kg |
| | Witepsol® | 14,3 kg |
| | Sojaöl | 9,2 kg |
| | Ethanol | 3,0 kg |

werden in einem Kneter bei 60° C homogen gemischt.

c) Auf einer Hartgelatinekapselmaschine mit einer Abfüllstation für Flüssigkeiten werden 500 mg der unter b) beschriebenen Füllmasse in eine Hartgelatinekapsel der Größe 0 abgefüllt. Anschließend wird auf derselben Maschine mit Hilfe einer Abfüllstation für Tabletten ein Preßling der unter a) beschriebenen Zusammensetzung in die Kapsel eingelegt und die Kapsel verschlossen.

**Beispiel 23**

Prednisolon-Tabletten:

| | |
|---|---|
| Prednisolon | 5 mg |
| 1,2-Diacyl-glycero-3-phosphocholin/$CaCl_2$ | 100 mg |
| Cellulose microcristallin | 60 mg |
| Aerosil® 200 | 10 mg |
| Natriumcarboxymethylstärke | 15 mg |

| | |
|---|---|
| Magnesiumstearat | 10 mg |

Herstellung analog Beispiel 13.

**Beispiel 24**

Dexamethason-Tabletten
(9-Fluor-16-methylprednisolon)

| | |
|---|---|
| Dexamethason | 0,5 mg |
| 1,2-Diacyl-glycero-3-phosphocholin/$CaCl_2$ | 50,0 mg |
| Cellulose microcristallin | 30,0 mg |
| Aerosil® 200 | 5,0 mg |
| Natriumcarboxymethylstärke | 4,5 mg |
| Magnesiumstearat | 10 mg |

Herstellung analog Beispiel 13.

**Beispiel 25**

Methotrexat-Tabletten

| | |
|---|---|
| Methotrexat | 2,5 mg |
| 1,2-Diacyl-glycero-3-phosphocholin/$CaCl_2$ | 100,0 mg |
| Microcristalline Cellulose | 50,0 mg |
| Aerosil® | 15,0 mg |
| Magnesiumstearat | 7,5 mg |

Herstellung analog Beispiel 13.

**Beispiel 26**

p-Aminosalicylsäure-Tabletten:

| | |
|---|---|
| p-Aminosalicylsaures Natrium $2H_2O$ | 400,0 mg |
| 1,2-Diacyl-glycero-3-phosphocholin/$CaCl_2$ | 100,0 mg |
| STA-RX® 1500 (Spezial-Maisstärke) | 45,0 mg |
| Hydroxypropylcellulose | 15,0 mg |
| Aerosil® | 10,0 mg |

Herstellung analog Beispiel 12.

**Patentansprüche**

1. Verwendung von 1,2 Diacylglycero-3-phosphocholin in dem 75 - 86 Gew.-% der Acylreste ungesättigte Fettsäurereste sind und ein Gemisch aus Fettsäureresten aus

10 - 20 Gew.-% Palmitinsäure
4 - 5 Gew.-% Stearinsäure
10 - 12 Gew.-% Ölsäure
62 - 68 Gew.-% Linolsäure
3 - 6 Gew.-% Linolensäure

sind, wobei die einzelnen Gehalte so ausgewählt sind, daß sie jeweils 100 % ergeben, zur Herstellung einer pharmazeutischen Zubereitung zur Behandlung von Erkrankungen im Magen-Darm-Bereich.

2. Verwendung nach Anspruch 1
dadurch gekennzeichnet,
daß der 1-Acylrest

22 - 26 Gew.-% Palmitinsäure
6 - 9 Gew.-% Stearinsäure

8 - 12 Gew.-% Ölsäure
50 - 54 Gew.-% Linolsäure
4 - 6 Gew.-% Linolensäure

und der 2-Acylrest

1 - 2 Gew.-% Palmitinsäure
0 - 1 Gew.-% Stearinsäure
8 - 12 Gew.-% Ölsäure
75 - 85 Gew.-% Linolsäure
5 - 8 Gew.-% Linolensäure

als Fettsäurerestgemisch enthält.

3. Verwendung nach den Ansprüchen 1 oder 2,
dadurch gekennzeichnet,
daß bis zu 20 Gew.-% bezogen auf Gesamtmenge Phospholipide eines oder mehrerer 1,2 Diacyl-glycerol-3-phosphate, wie
1,2 Diacyl-glycerol-3-phosphoethanolamin,
1,2 Diacyl-glycerol-3-phosphoinositol,
1,2 Diacyl-glycerol-3-phosphoserin,
1,2 Diacyl-glycerol-3-phosphoglycerol,

enthalten sind, wobei die Acylreste die in den Ansprüchen 1 und 2 angegebene Bedeutung besitzen.

4. Verwendung nach den Ansprüchen 1 - 3,
dadurch gekennzeichnet,
daß bei der Herstellung pharmazeutischen Zubereitung das spezielle 1,2 Diacyl-glycerol-3-phosphocholin in Wasser oder einem organischen Lösungsmittel gelöst oder emulgiert wird, Calziumchlorid, in Wasser oder einem Alkohol gelöst oder aufgeschlämmt, zugegeben wird, die Lösungsmittel abgezogen werden, das erhaltene Produkt nach üblichen Verfahren getrocknet, ggfs. gemahlen, und nach üblichen Verfahren zu oral einnehmbaren pharmazeutischen Zubereitungen verarbeitet wird.

## Claims

1. Use of 1.2-diacyl-glycero-3-phosphocholine in which 75 to 86 % by weight of the acyl radicals are unsaturated fatty acid radicals and a mixture of the fatty acid radicals of

10 - 20 % by weight palmitic acid
4 - 5 % by weight stearic acid
10 - 12 % by weight oleic acid
62 - 68 % by weight linoleic acid
3 - 6 % by weight linolenic acid,

the individual amounts being selected so that they always total 100 %, for the preparation of a pharmaceutical composition for treating gastrointestinal disorders.

2. Use of claim 1,
characterized in that,
contained in the 1-acyl radical are

22 - 26 % by weight palmitic acid
6 - 9 % by weight stearic acid
8 - 12 % by weight oleic acid
50 - 54 % by weight linoleic acid
4 - 6 % by weight linolenic acid,

and in the 2-acyl radical

1 - 2 % by weight palmitic acid
0 - 1 % by weight stearic acid
8 - 12 % by weight oleic acid
75 - 85 % by weight linoleic acid
5 - 8 % by weight linolenic acid,

as the mixture of the fatty acid radicals.

3. Use of claims 1 or 2
characterized by
comprising up to 20 % by weight, based on the total amount of phospholipids, of one or more 1.2-diacyl-glycero-3-phosphates, such as
1.2-diacyl glycero-3-phosphoethynol amine,
1.2-diacyl glycero-3-phosphoinositol,
1.2-diacyl glycero-3-phosphoserine
1.2-diacyl glycero-3-phosphoglycerol and
the acyl radicals having the meanings defined in claims 1 and 2.
4. Use of claims 1 - 3
characterized in that,
for the preparation of the pharmaceutical composition the specific 1.2-diacyl-glycero-3-phosphocholines are dissolved or emulsified in water or an organic solvent, calcium chloride is added after having been dissolved or suspended in water or an alcohol, the solvents removed, the resulting product dried by common methods and if desired ground and formed by common methods into orally administerable pharmaceutical compositions.

**Revendications**

1. Emploi de la diacyl-1,2 glycéro-3 phosphocholine dans laquelle 75 - 86 % en poids des radicaux acyle sont des radicaux acides gras non saturés et sont un mélange de radicaux acides gras composé de

10 - 20 % en poids d'acide palmitique
4 - 5 % en poids d'acide stéarique
10 - 12 % en poids d'acide oléique
62 - 68 % en poids d'acide linoléique
3 - 6 % en poids d'acide linolénique,

étant précisé que les différentes teneurs sont choisies de façon à donner chaque fois 100 %, pour fabriquer une préparation pharmaceutique pour le traitement de maladies de la région gastro-intestinale.
2. Emploi selon la revendication 1,
caractérisé
en ce que le radical acyle-1 contient comme mélange de radicaux acides gras

22 - 26 % en poids d'acide palmitique
6 - 9 % d'acide stéarique
8 - 12 % en poids d'acide oléique
50 - 54 % en poids d'acide linoléique
4 - 6 % en poids d'acide linolénique

et le radical acyle-2,

1 - 2 % en poids d'acide palmitique
0 - 1 % en poids d'acide stéarique
8 - 12 % en poids d'acide oléique
5 - 85 % en poids d'acide linoléique
5 - 8 % en poids d'acide linolénique
3. Emploi selon la revendication 1 ou 2,
caractérisé
en ce que sont contenus, jusqu'à 20 % en poids, rapporté à la la quantité totale de phospholipide, d'un ou plusieurs diacyl-1,2 glycéro-3 phosphates, tels que
diacyl-1,2 glycéro-3 phosphoéthanolamine,
diacyl-1,2 glycéro-3 phosphoinositol,
diacyl-1,2 glycéro-3 phosphosérine,
diacyl-1,2 glycéro-3 phosphoglycérol,
étant précisé que les radicaux acyle ont la signification indiquée dans les revendications 1 et 2.
4. Emploi selon les revendications 1-3,
caractérisé
en ce que, lors de la fabrication d'une préparation pharmaceutique, on dissout ou on émulsifie la diacyl-1,2 glycéro-3 phosphocholine spéciale dans de l'eau ou dans un solvant organique, on ajoute du chlorure de calcium, en solution ou en suspension dans l'eau ou dans un alcool, on élimine le solvant, on séche le produit obtenu selon les procédés habituels, éventuellement on le broie et on le traite selon les procédés habituels pour obtenir des préparations pharmaceutiques administrables par voie orale.